# EUROPEAN PATENT APPLICATION

(11) **EP 4 635 332 A1**
(43) Date of publication of application: **22.10.2025**
(21) Application number: 22968564.9
(22) Date of filing: 16.12.2022
(51) Int. Cl.: A24F 40/50, A24F 40/40, A24F 40/90

(54) **POWER SUPPLY UNIT FOR AEROSOL GENERATION DEVICE, AND AEROSOL GENERATION DEVICE**

(71) Applicant: Japan Tobacco Inc., Tokyo 105-6927 (JP)
(72) Inventor: KAWANAGO Hiroshi, Tokyo 130-8603 (JP); MINATO Junji, Tokyo 130-8603 (JP)
(74) Representative: Hoffmann Eitle
(86) International application number: PCT/JP2022/046480
(87) International publication number: WO 2024/127656

(57) **Abstract**

An inhalation device (100) comprises: a power source unit (111C) for supplying power to a heating unit (121C); a step-up DC/DC converter (82) and/or a heating switch (85); a charging IC (81); and a main board (50) for mounting the charging IC (81), a power source connecting portion (51), and the step-up DC/DC converter (82) and/or the heating switch (85). At least one of the step-up DC DC converter (82) and/or the heating switch (85) is disposed closer to the power source connecting portion (51) than the charging IC (81). A power source voltage measurement pin (Ps) of the charging IC (81) is connected by way of voltage measurement wiring (521) to a position closer to the power source connecting portion (51) than a power source connection pin (Ps) in power source wiring (520) joining the power source connecting portion (51) and a power source connection pin (Pb).

## Description

### TECHNICAL FIELD

The present disclosure relates to a power supply unit for an aerosol-generating device, and to an aerosol-generating device.

### BACKGROUND ART

Aerosol-generating devices generally heat an aerosol source by adjusting the power supplied from a power source to a predetermined power for heating by means of a power conversion device, and then supplying the adjusted power to a heater. Furthermore, aerosol-generating devices are configured to be capable of repeated recharging when the SOC of a power source has decreased.

For example, PTL 1 describes an aerosol-generating device in which the power of a power source is boosted by a DC/DC converter and supplied to a heater to thereby heat an aerosolforming article, and the DC/DC converter comprises a feedback pin for adjusting an output voltage.

### CITATION LIST

### PATENT LITERATURE

[PTL 1] JP 2020-518236 A

### SUMMARY OF INVENTION

### TECHNICAL PROBLEM

In an aerosol-generating device such as this, both the heating elements for providing heating to the aerosol source and a charging IC for controlling charging are preferably disposed close to the power source. Among the heating elements, the power conversion device and a heating switch carry an especially large current flow for heating, and power loss therefrom is therefore preferably eliminated. Meanwhile, the charging IC also preferably takes measurements close to the power source when measuring a power source voltage for controlling charging. That is to say, when the measurement point is remote from the power source, there is a proportional increase in wiring resistance and a drop in the accuracy of charging control.

The present disclosure provides a power supply unit for an aerosol-generating device capable of maintaining the accuracy of charging control while improving heating efficiency, and also provides an aerosol-generating device.

### SOLUTION TO PROBLEM

The present disclosure relates to:
a power supply unit for an aerosol-generating device, the power supply unit comprising: a power source for supplying power to a heating unit for heating an aerosol source;
a power conversion device for converting power from the power source and supplying heating power to the heating unit and/or a heating switch for controlling power supply to the heating unit;
a charging IC for receiving power from an external power source and performing control to supply charging power to the power source; and
a board for mounting the charging IC, a power source connecting portion supplied with power from the power source, and the power conversion device and/or the heating switch, wherein the charging IC comprises:
   a power source voltage measurement pin for measuring a voltage of the power source; and
   a power source connection pin to which the voltage of the power source is input,
   at least one of the power conversion device and/or the heating switch is disposed closer to the power source connecting portion than the charging IC, and
   the power source voltage measurement pin of the charging IC is connected by way of voltage measurement wiring to a position closer to the power source connecting portion than the power source connection pin in power source wiring joining the power source connecting portion and the power source connection pin.

The present disclosure furthermore relates to:
a power supply unit for an aerosol-generating device, the power supply unit comprising: a power source for supplying power to a heating unit for heating an aerosol source;
a power conversion device for converting power from the power source and supplying heating power to the heating unit and/or a heating switch for controlling power supply to the heating unit;
a charging IC for receiving power from an external power source and performing control to supply charging power to the power source; and
a board for mounting the charging IC, a power source connecting portion supplied with power from the power source, and the power conversion device and/or the heating switch, wherein the charging IC comprises:
   a power source voltage measurement pin for measuring a voltage of the power source; and
   a power source connection pin to which the voltage of the power source is input,
   a wiring distance between at least one of the power conversion device and/or the heating switch and the power source connecting portion is shorter than a wiring distance between the charging IC and the power source connecting portion, and
   the power source voltage measurement pin of the charging IC is connected by way of voltage measurement wiring to a position closer to the power source connecting portion than the power source connection pin in power source wiring joining the power source connecting portion and the power source connection pin.

The present disclosure furthermore relates to:
an aerosol-generating device comprising: a heating unit for heating an aerosol source;
a power source for supplying power to the heating unit;
a power conversion device for converting power from the power source and supplying heating power to the heating unit and/or a heating switch for controlling power supply to the heating unit;
a charging IC for receiving power from an external power source and performing control to supply charging power to the power source; and
a board for mounting the charging IC, a power source connecting portion supplied with power from the power source, and the power conversion device and/or the heating switch, wherein the charging IC comprises:
   a power source voltage measurement pin for measuring a voltage of the power source; and
   a power source connection pin to which the voltage of the power source is input,
   at least one of the power conversion device and the heating switch is disposed closer to the power source connecting portion than the charging IC, and
   the power source voltage measurement pin of the charging IC is connected by way of voltage measurement wiring to a position closer to the power source connecting portion than the power source connection pin in power source wiring joining the power source connecting portion and the power source connection pin.

### ADVANTAGEOUS EFFECTS OF INVENTION

The present disclosure makes it possible to maintain the accuracy of charging control while improving heating efficiency.

### BRIEF DESCRIPTION OF DRAWINGS

Fig. 1 is a schematic diagram schematically showing a first configuration example of an inhalation device (inhalation device 100A).
Fig. 2 is a schematic diagram schematically showing a second configuration example of an inhalation device (inhalation device 100B).
Fig. 3 is an overall oblique view of an inhalation device 100 constituting an embodiment of the inhalation device according to the present disclosure.
Fig. 4 is an oblique view of an internal unit 10 seen from the front right side.
Fig. 5 is an oblique view of the internal unit 10 seen from the front left side.
Fig. 6 is an exploded oblique view of the internal unit 10.
Fig. 7 is a cross-sectional oblique view of a heater assembly 30.
Fig. 8 is a block diagram showing electrical connections of main elements of the internal unit 10 in a simple manner.
Fig. 9 shows elements mounted on a front surface 501 of a main board 50.
Fig. 10 shows elements mounted on a rear surface 502 of the main board 50.
Fig. 11 shows a flow of power during heating in fig. 10.
Fig. 12 shows a flow of power during charging in fig. 10.
Fig. 13 shows main conductive tracks of a first conductive layer L1 to a tenth conductive layer L10 provided on the main board 50.

### DESCRIPTION OF EMBODIMENTS

An inhalation device, control method, and program according to an embodiment of the present disclosure will be described below with reference to the drawings. Two configuration examples (a first configuration example and a second configuration example) to which the configuration of the inhalation device according to the present disclosure can be applied will be described first of all. It should be noted that, hereinafter, identical or similar components will be assigned identical or similar reference signs, and descriptions thereof may be omitted or simplified, as appropriate.

### 1. Configuration example of inhalation device

An inhalation device is a device for generating a substance to be inhaled by a user. Hereinafter, the substance generated by the inhalation device will be described as being an aerosol. Alternatively, the substance generated by the inhalation device may be a gas.

### (1) First configuration example

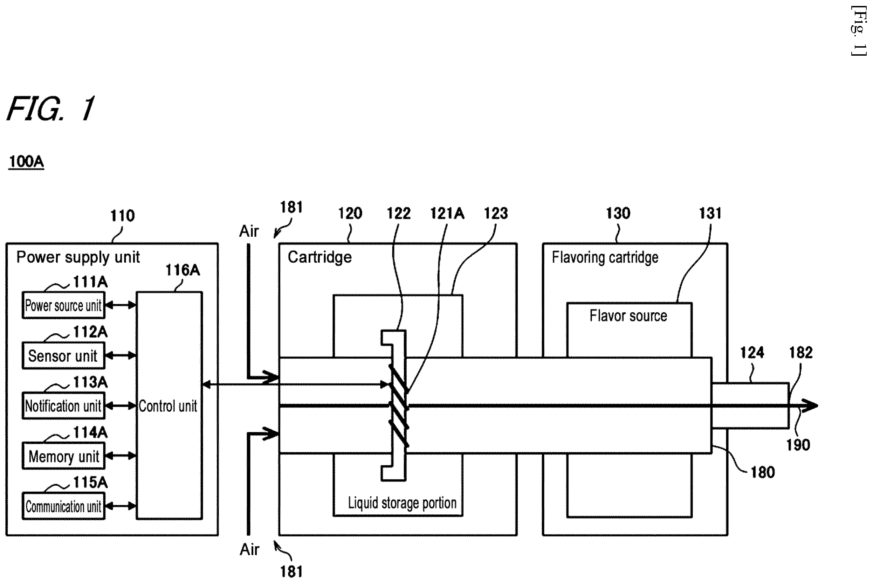

Fig. 1 is a schematic diagram schematically showing a first configuration example of an inhalation device. As shown in fig. 1, an inhalation device 100A according to this configuration example includes a power supply unit 110, a cartridge 120, and a flavoring cartridge 130. The power supply unit 110 comprises a power source unit 111A, a sensor unit 112A, a notification unit 113A, a memory unit 114A, a communication unit 115A, and a control unit 116A. The cartridge 120 includes a heating unit 121A, a liquid guiding portion 122, and a liquid storage portion 123. The flavoring cartridge 130 includes a flavor source 131 and a mouthpiece 124. An air flow path 180 is formed in the cartridge 120 and the flavoring cartridge 130.

The power source unit 111A stores electrical power. The power source unit 111A then supplies the electrical power to each component of the inhalation device 100A in accordance with control performed by the control unit 116A. The power source unit 111A may be configured, for example, by a rechargeable battery such as a lithium ion secondary battery.

The sensor unit 112A acquires various types of information relating to the inhalation device 100A. As an example, the sensor unit 112A is configured from a pressure sensor such as a capacitor microphone, a flow rate sensor or a temperature sensor, and so on, and acquires values associated with inhalation by a user. As another example, the sensor unit 112A is configured from an input device, such as a button or switch, for accepting input of information from the user.

The notification unit 113A notifies the user of information. The information notified to the user by the notification unit 113A includes, for example, a state of charge (SOC) indicating the state of charge of the power source unit 111A, a preheating time at the time of inhalation, and an inhalation-possible period, etc. The notification unit 113A can be configured by a light-emitting device which emits light, a display device which displays images, a sound output device which outputs sound, or a vibration device which vibrates, etc., for example.

The memory unit 114A stores various types of information for the operation of the inhalation device 100A. The memory unit 114A can be configured by a non-volatile storage medium such as a flash memory, for example.

The communication unit 115A is a communication interface capable of performing communication in accordance with any wired or wireless communication standard. Examples of communication standards that may be used include standards that employ Wi-Fi (registered trademark), Bluetooth (registered trademark), Bluetooth Low Energy (BLE) (registered trademark), Near-Field Communication (NFC), or Low Power Wide Area (LPWA), and so on.

The control unit 116A functions as an arithmetic processing device and a control device, and controls overall operation within the inhalation device 100A in accordance with various programs. The control unit 116A is realized by a central processing unit (CPU) or an electronic circuit such as a microprocessor, for example.

The liquid storage portion 123 stores an aerosol source. The aerosol source is atomized to generate an aerosol. The aerosol source is a polyhydric alcohol such as glycerol or propylene glycol, or a liquid such as water, for example. The aerosol source may include tobacco-derived or non-tobacco-derived flavor components. If the inhalation device 100A is a medical inhaler such as a nebulizer, the aerosol source may include a drug.

The liquid guiding portion 122 guides the aerosol source, which is the liquid stored in the liquid storage portion 123, from the liquid storage portion 123, and holds the aerosol source. The liquid guiding portion 122 is, for example, a wick formed by twisting a fibrous material such as glass fibers or a porous material such as a porous ceramic. In such a case, the aerosol source stored in the liquid storage portion 123 is guided by the capillary effect of the wick.

The heating unit 121A heats the aerosol source to atomize the aerosol source, thereby generating the aerosol. In the example shown in fig. 1, the heating unit 121A is configured as a coil wrapped around the liquid guiding portion 122. When the heating unit 121A generates heat, the aerosol source held in the liquid guiding portion 122 is then heated and atomized, generating the aerosol. The heating unit 121A generates heat when supplied with electricity from the power source unit 111A. As an example, electricity may be supplied to the heating unit 121A when the sensor unit 112A has detected that the user has started inhaling and/or that predetermined information has been input. The supply of electricity to the heating unit 121A may then be stopped when the sensor unit 112A has detected that the user has finished inhaling and/or that predetermined information has been input. Note that the inhalation action of the user on the inhalation device 100A is detectable, for example, based on pressure (internal pressure) exceeding a predetermined threshold in the inhalation device 100A detected by an inhalation sensor.

The flavor source 131 is a component for imparting a flavor component to the aerosol. The flavor source 131 may include tobacco-derived or non-tobacco-derived flavor components.

The air flow path 180 is a flow path for air to be inhaled by the user. The air flow path 180 has a tubular structure with an air inflow hole 181, which is an inlet for air into the air flow path 180, and an air outflow hole 182, which is an outlet for air from the air flow path 180, forming the two ends thereof. Along the air flow path 180, the liquid guiding portion 122 is disposed upstream (closer to the air inflow hole 181), and the flavor source 131 is disposed downstream (closer to the air outflow hole 182). Air flowing in through the air inflow hole 181 as the user inhales is mixed with the aerosol generated by the heating unit 121A and transported through the flavor source 131 to the air outflow hole 182, as shown by the arrow 190. When the mixed fluid of aerosol and air passes through the flavor source 131, the flavor component contained in the flavor source 131 is added to the aerosol.

The mouthpiece 124 is a member that is held in the user's mouth during inhalation. The air outflow hole 182 is disposed in the mouthpiece 124. The user holds the mouthpiece 124 in their mouth to make it possible to draw the mixed fluid of aerosol and air into the oral cavity.

A configuration example of the inhalation device 100A has been described above. The inhalation device 100A is, of course, not limited to the configuration described above, and various configurations may be adopted, such as those illustrated below as examples.

As an example, the inhalation device 100A need not include the flavoring cartridge 130. In such case, the cartridge 120 is provided with the mouthpiece 124.

As another example, the inhalation device 100A may include a plurality of types of aerosol sources. A plurality of types of aerosol generated from the plurality of types of aerosol sources may be mixed within the air flow path 180 to cause a chemical reaction, thereby generating yet more other types of aerosol.

Furthermore, the means for atomizing the aerosol source is not limited to heating provided by the heating unit 121A. For example, the means for atomizing the aerosol source may be vibration atomization or induction heating.

### (2) Second configuration example

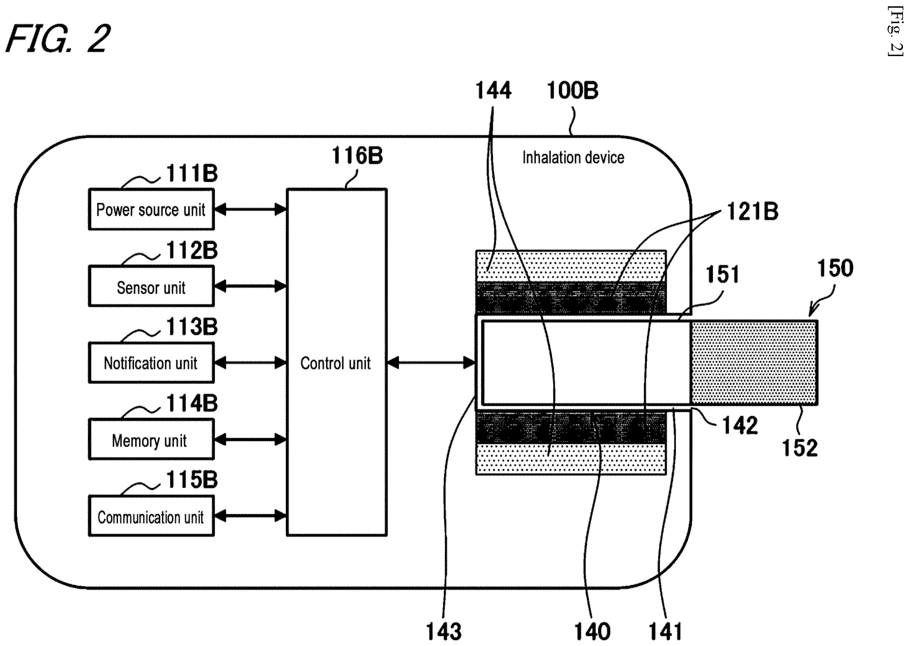

Fig. 2 is a schematic diagram schematically showing a second configuration example of the inhalation device. As shown in fig. 2, an inhalation device 100B according to this configuration example comprises a power source unit 111B, a sensor unit 112B, a notification unit 113B, a memory unit 114B, a communication unit 115B, a control unit 116B, a heating unit 121B, an accommodating portion 140, and a heat insulating portion 144. The power supply unit 110 accommodating the power source unit 111A, and the heating unit 121A were separate elements in the inhalation device 100A of the first configuration example, but the power source unit 111B and the heating unit 121B constitute a single piece in the inhalation device 100B of the second configuration example. That is, the inhalation device 100B of the second configuration example can also be said to have a power supply unit with a built-in heating unit.

The power source unit 111B, sensor unit 112B, notification unit 113B, memory unit 114B, communication unit 115B, and control unit 116B are each substantially the same as the corresponding component included in the inhalation device 100A of the first configuration example.

The accommodating portion 140 has an internal space 141, and holds a stick-type substrate 150 while accommodating a portion of the stick-type substrate 150 in the internal space 141. The accommodating portion 140 has an opening 142 allowing the internal space 141 to communicate with the outside, and accommodates the stick-type substrate 150 which has been inserted into the internal space 141 from the opening 142. For example, the accommodating portion 140 is a cylindrical body comprising the opening 142 and a bottom portion 143 serving as a bottom surface, and defines the columnar internal space 141. An air flow path for supplying air to the internal space 141 is connected to the accommodating portion 140. An air inflow hole, which is an inlet for air into the air flow path, is disposed in a side surface of the inhalation device 100, for example. An air outflow hole serving as an outlet for air from the air flow path to the internal space 141 is disposed in the bottom portion 143, for example.

The stick-type substrate 150 comprises a substrate portion 151 and a mouthpiece portion 152. The substrate portion 151 contains an aerosol source. The aerosol source comprises a tobacco-derived or non-tobacco-derived flavor component. If the inhalation device 100B is a medical inhaler such as a nebulizer, the aerosol source may include a drug. The aerosol source may be, for example, a liquid such as water and polyhydric alcohols such as glycerol and propylene glycol comprising the tobacco-derived or non-tobacco-derived flavor component, or else may be a solid comprising the tobacco-derived or non-tobacco-derived flavor component. In a state in which the stick-type substrate 150 is held in the accommodating portion 140, at least part of the substrate portion 151 is accommodated in the internal space 141, and at least part of the mouthpiece portion 152 protrudes from the opening 142. Then, when the user holds the mouthpiece portion 152 protruding from the opening 142 in their mouth and inhales, air flows into the internal space 141 via the air flow path, which is not illustrated on the drawings, and reaches the inside of the user's mouth together with the aerosol generated from the substrate portion 151.

In the example shown in fig. 2, the heating unit 121B has a film-like form and is arranged so as to cover the outer circumference of the accommodating portion 140. Then, when the heating unit 121B generates heat, the substrate portion 151 of the stick-type substrate 150 is heated from the outer circumference, generating the aerosol.

The heat insulating portion 144 prevents heat transfer from the heating unit 121B to other components. For example, the heat insulating portion 144 is configured by a vacuum insulating material or an aerogel insulating material, etc.

A configuration example of the inhalation device 100B has been described above. The inhalation device 100B is, of course, not limited to the configuration described above, and various configurations may be adopted, such as those illustrated below as examples.

As one example, the heating unit 121B may have a blade-like form and may be arranged so as to protrude into the internal space 141 from the bottom portion 143 of the accommodating portion 140. In that case, the blade-like heating unit 121B is inserted into the substrate portion 151 of the stick-type substrate 150 and heats the substrate portion 151 of the stick-type substrate 150 from the inside. As another example, the heating unit 121B may be arranged so as to cover the bottom portion 143 of the accommodating portion 140. Furthermore, the heating unit 121B may be configured from a combination of two or more from among a first heating unit covering the outer circumference of the accommodating portion 140, a blade-like second heating unit, and a third heating unit covering the bottom portion 143 of the accommodating portion 140.

As another example, the accommodating portion 140 may comprise an opening/closing mechanism such as a hinge for opening/closing part of an external casing that forms the internal space 141. By opening/closing the external casing, the accommodating portion 140 may then receive and grip the stick-type substrate 150 that has been inserted into the internal space 141. In this case, the heating unit 121B may be provided on the gripping part of the accommodating portion 140, and may heat the stick-type substrate 150 while pressing the same.

Furthermore, the means for atomizing the aerosol source is not limited to heating provided by the heating unit 121B. For example, the means for atomizing the aerosol source may be induction heating. In this case, the inhalation device 100B comprises at least an electromagnetic induction source such as a coil for generating a magnetic field, instead of the heating unit 121B. A susceptor which generates heat by means of induction heating may be provided in the inhalation device 100B, or may be contained in the stick-type substrate 150.

The inhalation device 100B may further include the heating unit 121A, the liquid guiding portion 122, the liquid storage portion 123, and the air flow path 180 according to the first configuration example, and the air flow path 180 may supply air to the internal space 141. In this case, the mixed fluid of aerosol and air generated by the heating unit 121A flows into the internal space 141 and is further mixed with the aerosol generated by the heating unit 121B, and reaches the oral cavity of the user.

### 2. Configuration example of inhalation device according to the present disclosure

Next, an embodiment of the inhalation device (hereinafter referred to as the inhalation device 100) applying the configuration of the inhalation device of the present disclosure is described in relation to the inhalation device 100B of the second configuration example previously described. Note that although the specific description is omitted, some of the configuration of the inhalation device 100 elaborated below can also be applied to the inhalation device 100A of the first configuration example.

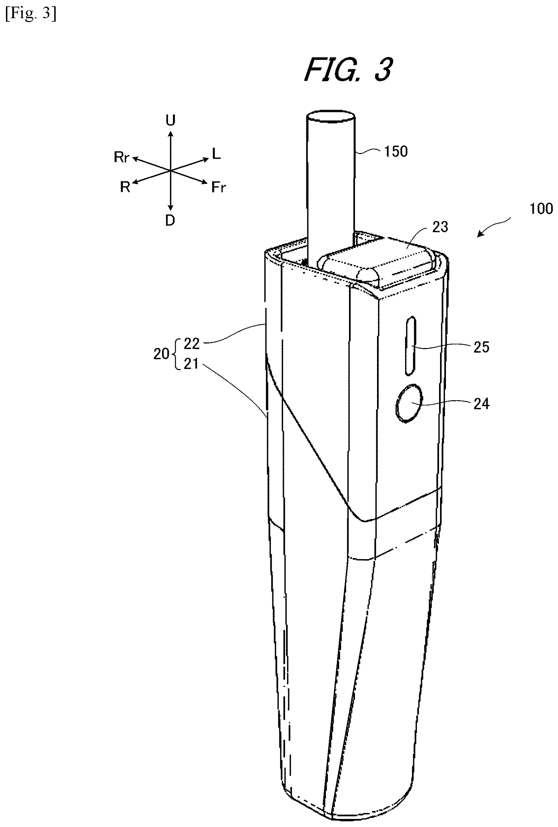

Fig. 3 is an overall oblique view of the inhalation device 100. In the following, in the inhalation device 100, the insertion and removal direction of the stick-type substrate 150 relative to the inhalation device 100 is defined as the vertical direction, the sliding movement direction of a shutter 23 described below is defined as the front-rear direction, and the direction perpendicular to the vertical direction and the front-rear direction is defined as the left-right direction. Also, as shown in the figures, Fr is the front, Rr is the rear, L is the left side, R is the right side, U is up, and D is down.

The inhalation device 100 is preferably sized to fit in the hand, for example, having a rod shape. For example, the user holds the inhalation device 100 in one hand, with fingertips in contact with surfaces of the inhalation device 100. Note that the shape of the inhalation device 100 is not limited to a rod shape, but can be any shape (e.g., a rounded substantially cuboid shape or an ovoid shape).

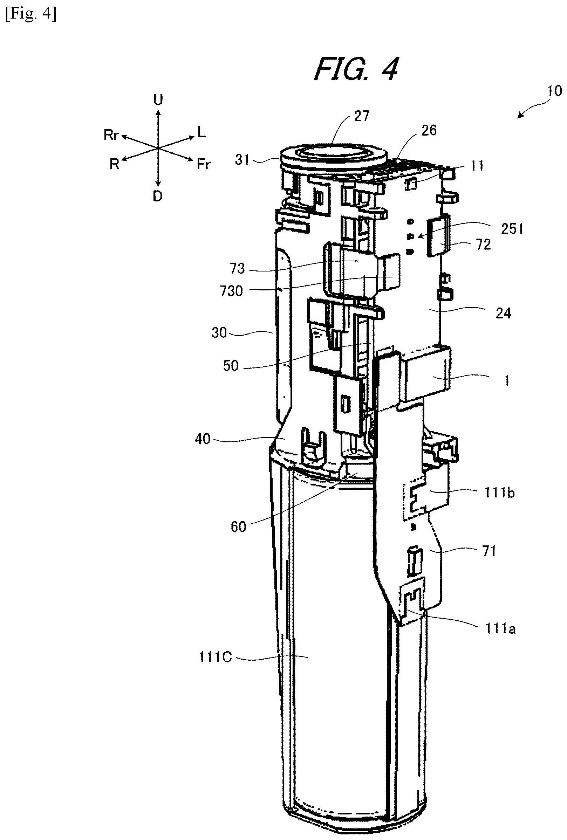

The inhalation device 100 comprises an internal unit 10 (see fig. 4-6), and a case 20 that constitutes the external appearance of the inhalation device 100. The case 20 has a lower case 21 and an upper case 22. A portion of the internal unit 10 is accommodated in the lower case 21, and the entire internal unit 10 is accommodated in the case 20 by covering the lower case 21 with the upper case 22 from above.

The upper face of the inhalation device 100 is provided with an opening 27 (see fig. 4 to 6) for inserting and removing the stick-type substrate 150, and a shutter 23 that can slide in the front-rear direction. The opening 27 is arranged on the rear side of the upper face of the inhalation device 100. The shutter 23 selectively takes an open state (front-side position) that allows insertion and removal of the stick-type substrate 150 by opening the opening 27, and a closed state (rear-side position) that closes the opening 27 by positioning the shutter 23 above the opening 27. When inserting the stick-type substrate 150 into the opening 27, the user sets the shutter 23 to the open state.

A shutter detection sensor 11 (see fig. 4) is provided near the shutter 23. The shutter detection sensor 11 detects whether or not the shutter 23 is in the open state. The shutter detection sensor 11 is an example of the sensor unit 112B of the inhalation device 100B of fig. 2.

Additionally, the upper surface of the inhalation device 100 is provided with a USB (Universal Serial Bus) port 26 (see fig. 4) arranged adjacent to the opening 27. In the open state described above, the shutter 23 blocks the USB port 26. On the other hand, in the closed state described above, the shutter 23 does not block the USB port 26 and the USB port 26 is open. The USB port 26 is configured to be electrically connectable with an external power source (not shown in the drawings) capable of supplying power to charge a power source unit 111C (see fig. 4). The USB port 26 is, for example, a receptacle in which a partnering plug can be inserted. As an example, in this embodiment, the USB port 26 is a USB Type-C receptacle.

An operation unit 24 and a light-emitting unit 25 are provided on the front face of the inhalation device 100. The operation unit 24 is arranged below the light-emitting unit 25. More specifically, the operation unit 24 and the light-emitting unit 25 are components of the internal unit 10 accommodated in the case 20, and a part of the operation unit 24 and the light-emitting unit 25 is configured to be exposed from an opening formed on the front face of the case 20. The light-emitting unit 25 is an example of the notification unit 113B of the inhalation device 100B shown in fig. 2.

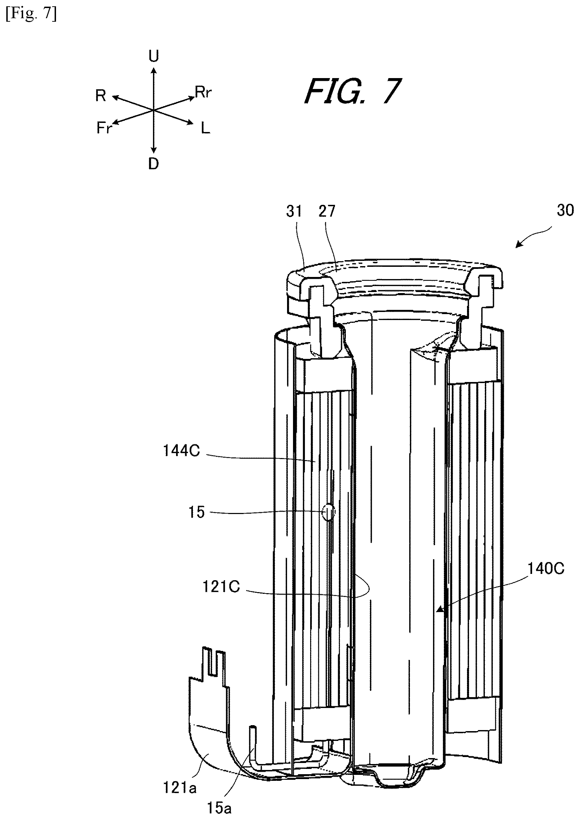

The operation unit 24 is a button-type switch that can be operated by a user, and is an input device for receiving input of information from a user. The operation unit 24 is connected to a main board 50 which will be described later (see fig. 4 to 6). The user pressing the operation unit 24, for example, activates a Micro Controller Unit (MCU) 1 (see fig. 4-6) or a heating unit 121C (see fig. 7). Note that the MCU 1 functions as the control unit 116B in the inhalation device 100B. In addition to the function of the control unit 116B in the inhalation device 100B, the MCU 1 may also incorporate the function of the communication unit 115B. Furthermore, the MCU 1 may be composed of a single IC or two or more ICs. For example, control of discharge to the heating unit 121C and control of charging of the power source unit 111C may be performed by a single IC or by separate ICs.

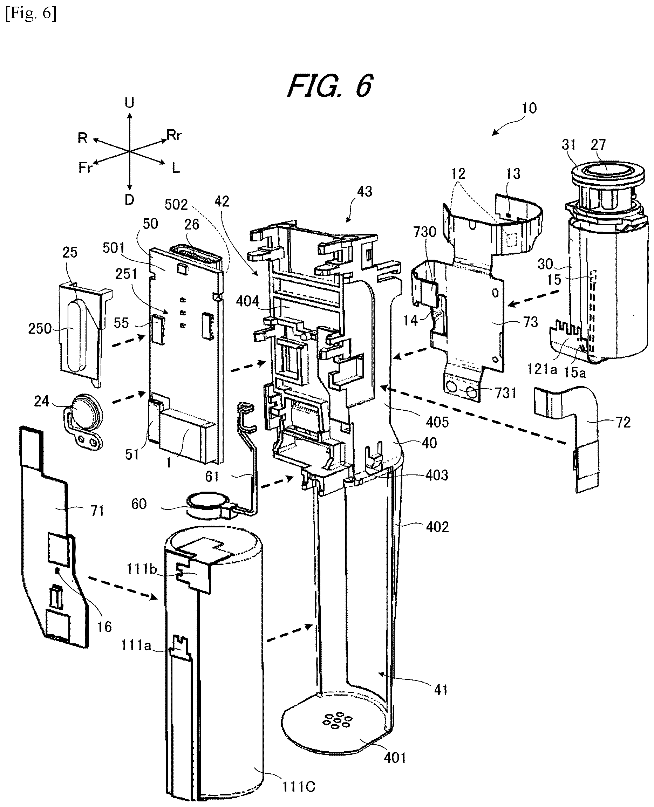

The light-emitting unit 25 is configured by a light-emitting device such as a light-emitting diode (LED), for example. More specifically, the light-emitting unit 25 includes multiple LEDs 251 (see fig. 6) provided on the main board 50, and a transparent cover 250 that covers the multiple LEDs 251 and transmits the light of the multiple LEDs 251. A portion of the transparent cover 250 is exposed through an opening formed in the front face of the case 20. In this embodiment, for example, it is assumed that the multiple LEDs 251 are configured to be capable of emitting light in a plurality of colors, including blue, yellow and red. Note that any number of light-emitting elements may be set, for example there may be one light emitting element in the light-emitting unit 25.

The light-emitting unit 25 emits light in a predetermined light-emitting mode by a command from the MCU 1 to notify the user of predetermined information. Here, the light-emitting mode may be, for example, a color of light emission, but this is not limiting, and it may be, for example, the intensity of illumination (in other words luminance) or an illumination pattern (e.g., flashing at predetermined time intervals), etc. Also, the predetermined information is, for example, operating information indicating whether the inhalation device 100 is powered on or not.

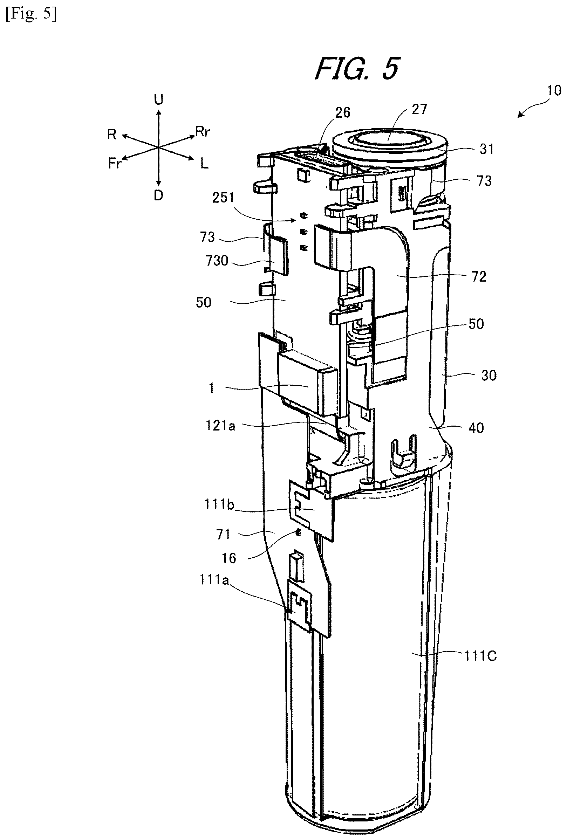

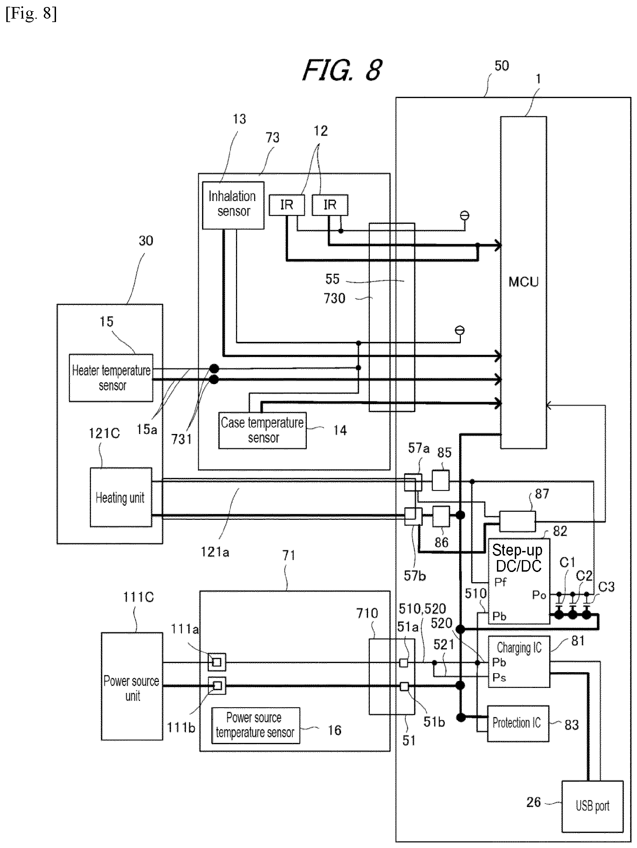

The internal unit 10 of the inhalation device 100 according to this embodiment will be described next with reference to fig. 4-6. Fig. 4 is an oblique view of the internal unit 10 seen from the front right side; fig. 5 is an oblique view of the internal unit 10 seen from the front left side; fig. 6 is an exploded oblique view of the internal unit 10; fig. 7 is a cross-sectional oblique view of a heater assembly 30; and fig. 8 is a block diagram showing electrical connections of main elements of the internal unit 10 in a simple manner. Note that the internal unit 10 constitutes the inhalation device 100 from which the case 20 and the shutter 23 have been removed.

The internal unit 10 comprises a chassis 40, the main board 50, a vibration device 60, a heater assembly 30, the power source unit 111C, a power source board 71, a peripheral flexible printed circuit (FPC) 72, a sensor FPC 73, and various sensors. The power source board 71, the peripheral FPC 72 and the sensor FPC 73 are flexible circuit boards. The flexible circuit board is flexible and comprises conduction wiring and/or signal wiring and allows mounting of electronic components (elements) such as resistors and chips. The flexible circuit board is generally set with a thickness of 100 µm-600 µm. The power source board 71 may be a flexible circuit board, a rigid board as described below, or a combination of a flexible board and a rigid board, but the example of a flexible circuit board is described here.

### Chassis

As shown in the exploded oblique view of fig. 6, the chassis 40 comprises: a power source holding portion 41 for holding the power source unit 111C; a board holding portion 42 for holding the main board 50; and a heater holding portion 43 for holding the heater assembly 30. The power source holding portion 41 is positioned at a lower portion of the chassis 40, and the board holding portion 42 and heater holding portion 43 are positioned on an upper portion of the chassis 40.

The power source holding portion 41 has a cylindrical shape with a portion of the side cut out, in other words a substantially semi-cylindrical shape. The power source holding portion 41 has a bottom wall portion 401, a side wall portion 402 having a circular arc shape and standing upright from the bottom wall portion 401, and an upper wall portion 403 provided at the upper end of the side wall portion 402. The power source unit 111C is arranged in a space surrounded by the bottom wall portion 401, the side wall portion 402 and the upper wall portion 403.

The board holding portion 42 is provided on a vertical wall portion 404 extending upward from the upper wall portion 403 of the power source holding portion 41. The board holding portion 42 is provided on one side (here on the front side) of the vertical wall portion 404 in the front-rear direction, and holds the main board 50.

The heater holding portion 43 is provided on the opposite side to (here on the rear side of) the board holding portion 42 of the vertical wall portion 404 in the front-rear direction. The heater holding portion 43 has a space surrounded by the vertical wall portion 404, a pair of left and right wall portions 405 extending from the vertical wall portion 404 in a front-rear direction, and an upper face of the upper wall portion 403 of the power source holding portion 41, and the heater assembly 30 is arranged in this space.

### Main board

The main board 50 is a rigid board with a plurality of electronic components (elements) mounted on both sides. The rigid board is not flexible and is generally set with a thickness of 300 µm-1600 µm. The MCU 1, LEDs 251, a charging IC (integrated circuit) 81, a step-up DC/DC converter 82, a protection IC 83, heating switches 85, 86, and an operational amplifier 87, etc. are mounted on the main board 50. The main board 50 is held in the board holding portion 42 of the chassis 40 so that the element mounting surface is oriented in the front-rear direction.

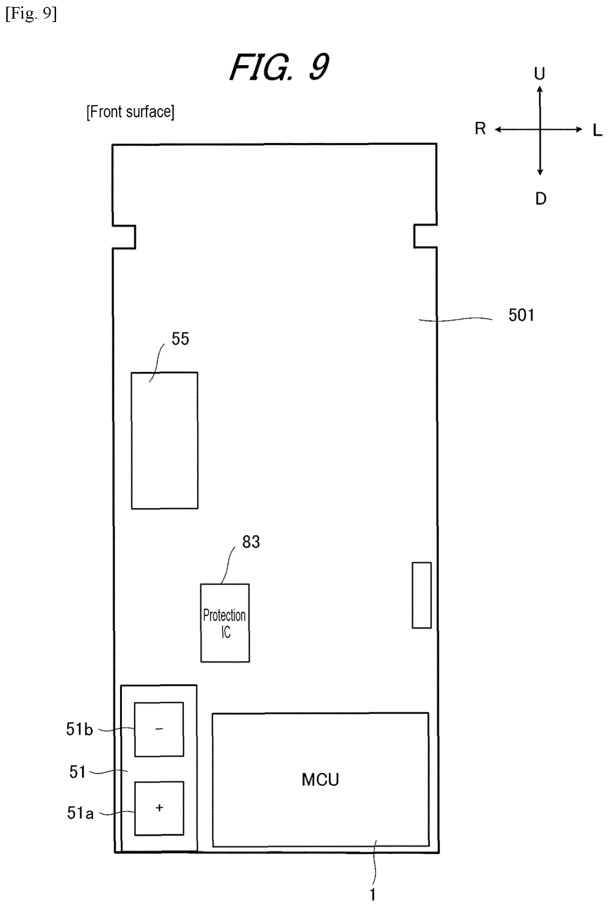

Fig. 9 shows elements mounted on a front surface 501 of a main board 50.

As shown in fig. 9, a power source connecting portion 51 which electrically connects to the power source unit 111C at the right-hand end is provided in a lower region on the front surface 501 of the main board 50. A positive electrode-side connecting portion 51a is provided below a negative electrode-side connecting portion 51b of the power source connecting portion 51. The power source connecting portion 51 is electrically connected to the power source unit 111C via the power source board 71. The power source unit 111C is a cylindrical lithium ion secondary battery, and is an example of the power source unit 111B of the inhalation device 100B of fig. 2.

As shown in fig. 6, the power source unit 111C is provided with a positive electrode tab 111a and a negative electrode tab 111b. The power source unit 111C is arranged in the power source holding portion 41 of the chassis 40 so that the positive electrode tab 111a and the negative electrode tab 111b are arranged to the front. The power source board 71 is arranged in front of the power source unit 111C and the main board 50, and extends in the vertical direction. The power source board 71 is connected to the positive electrode tab 111a and the negative electrode tab 111b of the power source unit 111C and is also connected to the power source connecting portion 51 of the main board 50. Power from the power source unit 111C is transmitted to the main board 50 through a conductive track formed on the power source board 71, and is supplied to each electronic component, such as the step-up DC/DC converter 82 and the protection IC 83, for example. The power source board 71 is also provided with a power source temperature sensor 16. The power source temperature sensor 16 is a temperature sensor for detecting the temperature of the power source unit 111C. The power source temperature sensor 16 is, for example, a thermistor. The power source temperature sensor 16 is an example of the sensor unit 112B of the inhalation device 100B of fig. 2.

Returning to fig. 9, the MCU 1 is mounted in a lower region on the front surface 501 of the main board 50, to the left of the power source connecting portion 51, and the protection IC 83 is mounted above the MCU 1, in the vicinity of the power source connecting portion 51. Referring also to fig. 8, the protection IC 83 is intended to protect the power source unit 111C by stopping charging or discharging of the power source unit 111C in the event of overcharging or overdischarging when the power source unit 111C is charging or discharging.

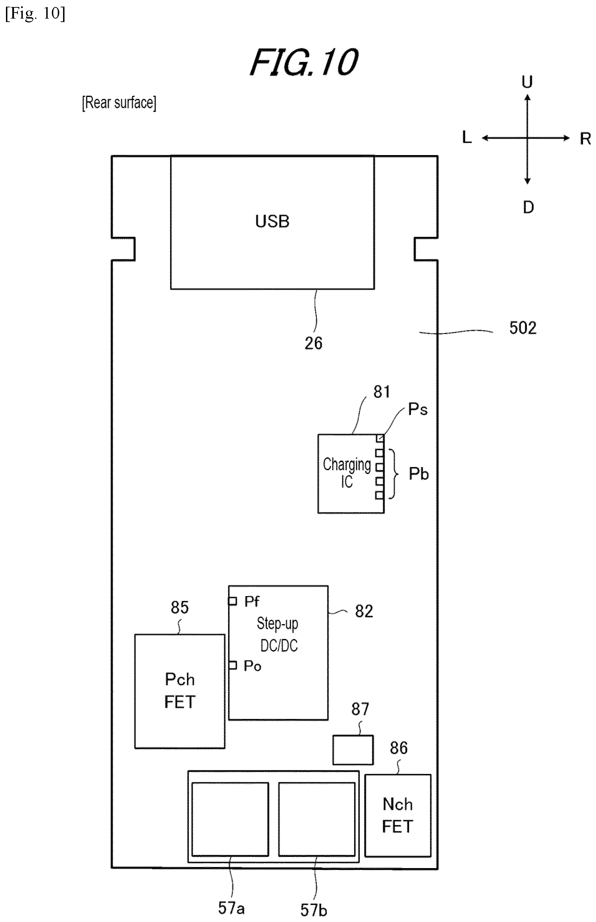

Fig. 10 shows elements mounted on a rear surface 502 of the main board 50.

As shown in fig. 10, the USB port 26 is provided in an upper region on the rear surface 502 of the main board 50. The USB port 26 is electrically connected to the charging IC 81 by wiring formed on the main board 50.

The charging IC 81 is mounted toward the right in a central region of the rear surface 502 of the main board 50, heater connecting portions 57a, 57b are provided in the center of a lower region, and a low-potential-side heating switch 86 (Nch FET in the drawing) is mounted to the right of the heater connecting portions 57a, 57b in the lower region. Furthermore, a highpotential-side heating switch 85 (Pch FET in the drawing), the step-up DC/DC converter 82, and the operational amplifier 87 are mounted in that order from the left, between the charging IC 81 and the heater connecting portions 57a, 57b, on the rear surface 502 of the main board 50. The charging IC 81 performs charging control to supply the power input from the USB port 26 to (charge) the power source unit 111C. The step-up DC/DC converter 82 steps up the voltage of the power supplied from the power source unit 111C, to generate power to supply to the heating unit 121C (see fig. 7) via the heating switches 85, 86. The heating switches 85, 86 are FETs (field effect transistors), for example. By providing the power source connecting portion 51 on the front surface 501 while the heating elements such as the step-up DC/DC converter 82 and the heating switches 85, 86 are mounted on the rear surface 502 in this way, the heating elements and the power source connecting portion 51 can be arranged closer together. Since the protection IC 83 is prone to errors at high temperatures, a deterioration in control accuracy can be suppressed by mounting the protection IC 83 on the opposite surface to the heating elements which are heat-generating elements.

A board connecting portion 121a extending from below the heater assembly 30 is connected to the heater connecting portions 57a, 57b to provide power to the heating unit 121C of the heater assembly 30. As a result, power from the power source unit 111C is supplied to the heating unit 121C of the heater assembly 30 via the main board 50.

As shown in fig. 8, the operational amplifier 87 is connected to the heater connecting portions 57a, 57b. The operational amplifier 87 amplifies and outputs a difference in a voltage input to an inverting input terminal and a voltage input to a non-inverting input terminal, in order to measure a resistance value of the heating unit 121C, although this will not be described in detail. The MCU 1 acquires the temperature of the heating unit 121C on the basis of the voltage input from the operational amplifier 87. By arranging the operational amplifier 87 on the same surface as the heating elements, it is thus possible to increase the signal-to-noise ratio, i.e., SNR, for measuring the heater resistance value. In addition, the operational amplifier 87 may employ a zero drift amplifier in order to reduce drift errors caused by heat generation.

### Vibration Device

The vibration device 60 is configured by a vibrating element such as a vibrating motor, for example. As shown in fig. 6, the vibration device 60 is arranged in the power source holding portion 41 of the chassis 40 between the upper face of the power source unit 111C and the upper wall portion 403. A lead wire 61 of the vibration device 60 is connected to the peripheral FPC 72. The vibration device 60 vibrates in a predetermined vibration mode by a command from the MCU 1, to notify the user of predetermined information. For example, at the start or end of heating of the stick-type substrate 150, the vibration device 60 vibrates in a predetermined vibration mode to notify the user of the start or end of heating. The vibration device 60 is an example of the notification unit 113B of the inhalation device 100B of fig. 2.

### Heater assembly

As shown in fig. 7, the heater assembly 30 comprises the heating unit 121C, an accommodating portion 140C, and a heat insulating portion 144C. The heating unit 121C is, for example, a film heater, and is wound around the outer circumference of the accommodating portion 140C. Furthermore, the heating unit 121C and the board connecting portion 121a may be configured by a single heater FPC.

The heater assembly 30 is also provided with a stick guide 31. The stick guide 31 is provided on an upper portion of the heater assembly 30 and guides insertion/removal of the stick-type substrate 150 into/from the accommodating portion 140C. The stick guide 31 is a cylindrical member with an opening 27 and constitutes part of the accommodating portion 140C.

Furthermore, the heater assembly 30 is provided with a heater temperature sensor 15 capable of detecting the temperature of the heating unit 121C. More specifically, the heater temperature sensor 15 is provided in contact with or in proximity to the heating unit 121C between the heating unit 121C and the heat insulating portion 144C. The heater temperature sensor 15 is, for example, a thermistor.

### Sensor FPC

As shown in fig. 6, the sensor FPC 73 is arranged in the heater holding portion 43 between the vertical wall portion 404 and the heater assembly 30. The sensor FPC 73 is equipped with a stick detection sensor 12, an inhalation sensor 13, and a case temperature sensor 14. The stick detection sensor 12, the inhalation sensor 13, and the case temperature sensor 14 are examples of the sensor unit 112B of the inhalation device 100B of fig. 2.

The stick detection sensor 12 is a sensor capable of detecting the stick-type substrate 150 accommodated in the accommodating portion 140C. In this embodiment, the stick detection sensor 12 is an optical sensor capable of detecting the stick-type substrate 150 based on the amount of reflected light from the light irradiated onto the accommodating portion 140C. Here, amount of light is a concept that includes luminous flux, illuminance, luminous emittance, brightness, luminance, and so on. The optical sensor is an infrared ray (IR) sensor, for example.

The inhalation sensor 13 is a sensor that detects a user puffing action (inhalation action). The inhalation sensor 13 comprises, for example, a capacitor microphone, a pressure sensor, a thermistor, or the like. The inhalation sensor 13 is provided in proximity to the stick guide 31 in the sensor FPC 73.

The case temperature sensor 14 is a sensor that detects the temperature of the case 20. The case temperature sensor 14 is, for example, a thermistor. The case temperature sensor 14 is arranged in the sensor FPC 73 next to the inner surface of the case 20.

The sensor FPC 73 is also provided with a heater temperature sensor connecting portion 731 connecting to the heater temperature sensor 15 of the heater assembly 30. The heater temperature sensor connecting portion 731 is provided on a lower portion of the sensor FPC 73. More specifically, a lead wire 15a is connected to the heater temperature sensor 15, and the heater temperature sensor connecting portion 731 is connected to the lead wire 15a extending from underneath the heater assembly 30.

The stick detection sensor 12, inhalation sensor 13, case temperature sensor 14, and heater temperature sensor connecting portion 731 are connected to a board connecting portion 730 via conductive tracks formed on the sensor FPC 73. The board connecting portion 730 is connected to a sensor FPC connecting portion 55 provided in a central region of the front surface 501 of the main board 50. As a result, detection results of the sensors are output to the MCU 1 and other components mounted on the main board 50.

In the inhalation device 100 configured in this way, when the open state of the shutter 23 is detected by the shutter detection sensor 11 and the stick-type substrate 150 is detected by the stick detection sensor 12, the MCU 1 starts heating by the heating unit 121C. When a user holds the mouthpiece portion 152 of the stick-type substrate 150 in their mouth and inhales, aerosol is supplied into the user's mouth from the aerosol source of the stick-type substrate 150 heated by the heating unit 121C. The inhalation sensor 13 detects the number of inhalations, and the MCU 1 stops heating after a predetermined number of inhalations or after a predetermined time has elapsed. During heating of the inhalation device 100, the case temperature sensor 14, the heater temperature sensor 15, and the power source temperature sensor 16 measure temperatures, and the MCU 1 stops or inhibits heating by the heating unit 121C if it is determined that there is abnormal heating. Furthermore, the user is able to check the SOC of the power source unit 111C, for instance, by operating the operation unit 24, for example. The light-emitting unit 25 (LEDs 251) and the vibration device 60 notify the user of various information such as the SOC of the power source unit 111C, error displays, and so on. If the SOC of the power source unit 111C decreases, the user can connect an external power source to the USB port 26 to charge the power source unit 111C.

### Details of main board

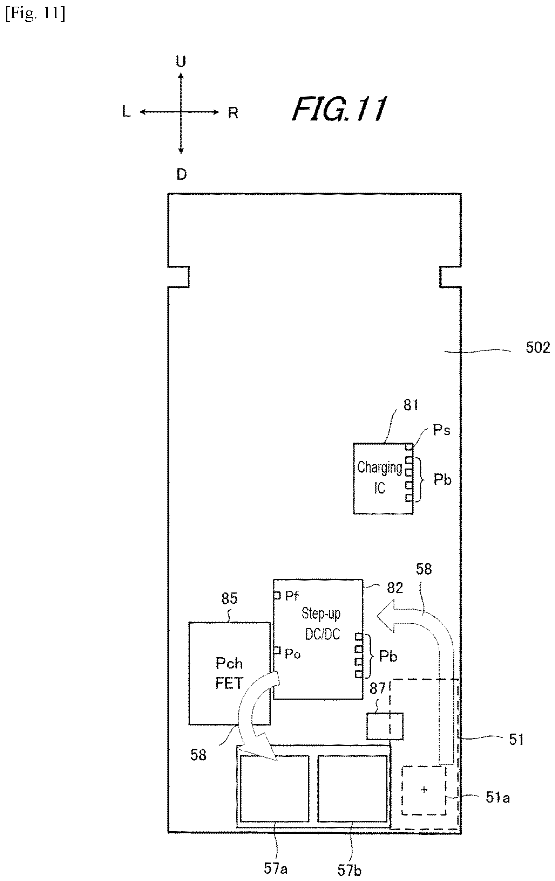

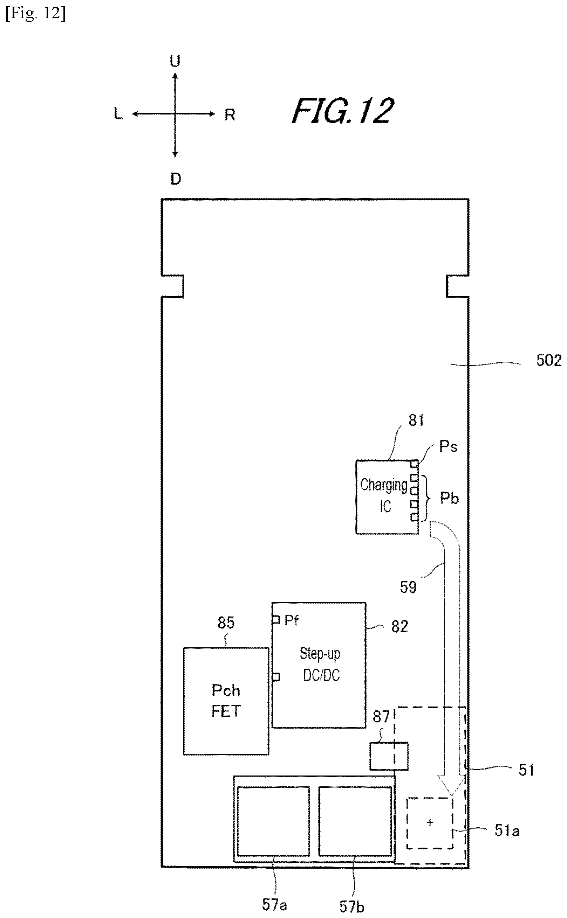

Details of the main board 50 will be described below with reference to fig. 8 and 11-13. Fig. 11 shows the flow of power during heating in fig. 10, and fig. 12 shows the flow of power during charging in fig. 10. In fig. 11 and 12, the main board 50 is viewed from the rear surface 502 side, and the power source connecting portion 51 (positive electrode-side connecting portion 51a) provided on the front surface 501 is denoted by broken lines. It should be noted that fig. 9 shows the power source connecting portion 51 with the front surface 501 facing forward, so the position of the power source connecting portion 51 appears in reverse in fig. 11 and 12 where the power source connecting portion 51 is shown with the rear surface 502 facing forward. Furthermore, the heating switch 86 has been omitted from fig. 11 and 12 because it lies over the power source connecting portion 51.

The positional relationships of elements mounted on the main board 50 are as described above, but when these elements are seen in relation to the power source connecting portion 51, the heating elements such as the step-up DC/DC converter 82 and the heating switches 85, 86 are disposed closer to the power source connecting portion 51 than the charging IC 81 which is a charging element. In terms of wiring distances, the elements are arranged so that the distance of wiring joining the power source connecting portion 51 and the heating elements such as the step-up DC/DC converter 82 and the heating switches 85, 86 is shorter than the distance of wiring joining the power source connecting portion 51 and the charging IC 81 which is a charging element.

The white arrow 58 in fig. 11 shows the flow of power during discharging, with power from the power source unit 111C, which is input to power source connection pins Pb from the positive electrode-side connecting portion 51a of the power source connecting portion 51, being supplied to the heater connecting portion 57a in a counterclockwise flow through a heating unit connection pin Po of the step-up DC/DC converter 82 and the high potential-side heating switch 85.

The white arrow 59 in fig. 12 shows the flow of power during charging, with power which is supplied from the USB port 26 to the charging IC 81 flowing downward from the power source connection pins Pb of the charging IC 81, and flowing through the positive electrode-side connecting portion 51a of the power source connecting portion 51.

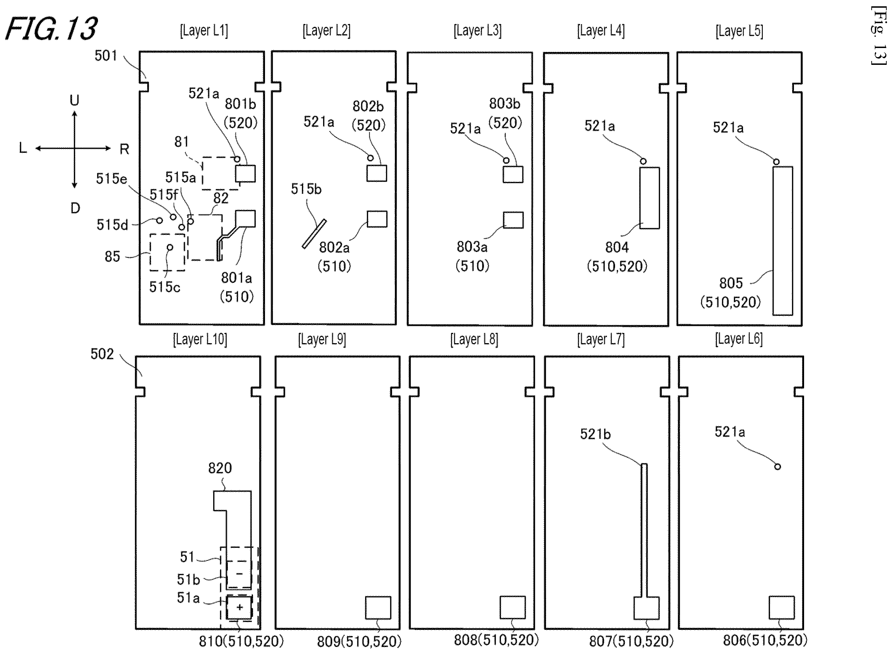

Fig. 13 shows main conductive tracks of a first conductive layer L1 to a tenth conductive layer L10 provided on the main board 50. In fig. 13, the first conductive layer L1 is a conductive track exposed on the rear surface 502, and the tenth conductive layer L10 is a conductive track exposed on the front surface 501. The charging IC 81, the step-up DC/DC converter 82, and the heating switch 85, etc. which are mounted on the rear surface 502 are connected to the first conductive layer L1. Portions of the tenth conductive layer L10 (lower end portions of conductive tracks 810, 820 which will be described later) constitute the positive electrode-side connecting portion 51a and the negative electrode-side connecting portion 51b.

The conductive track 810 of the tenth conductive layer L10 forming the positive electrode-side connecting portion 51a is connected to conductive tracks 809-805 of the ninth conductive layer L9 to the fifth conductive layer L5 which are formed at the same position. The conductive track 805 of the fifth conductive layer L5 extends upward over the board and is connected to the conductive track 804 of the fourth conductive layer L4. The conductive track 804 of the fourth conductive layer L4 is roughly half the vertical length of the conductive track 805, a lower portion thereof is connected to a conductive track 803a of the third conductive layer L3, and an upper portion thereof is connected to a conductive track 803b of the third conductive layer L3. It should be noted that connections between conductive tracks of different conductive layers are made through vias which are not depicted.

The conductive track 803a of the third conductive layer L3 is connected to the power source connection pins Pb (see fig. 11) of the step-up DC/DC converter 82 by way of a conductive track 802a of the second conductive layer L2 and a conductive track 801a of the first conductive layer L1 which are formed at the same position. That is to say, the conductive tracks 801a-803a and 804-810 are parts of the white arrow 58 in fig. 11, constituting the power source wiring 510 during discharging.

The conductive track 803b of the third conductive layer L3 is connected to the power source connection pins Pb (see fig. 11) of the charging IC 81 by way of a conductive track 802b of the second conductive layer L2 and a conductive track 801b of the first conductive layer L1 which are formed at the same position. That is to say, the conductive tracks 801b-803b and 804-810 are parts of the white arrow 59 in fig. 12, constituting the power source wiring 520 during charging.

The power source wiring 520 joining the positive electrode-side connecting portion 51a and the power source connection pins Pb of the charging IC 81, and the power source wiring 510 joining the positive electrode-side connecting portion 51a and the power source connection pins Pb of the step-up DC/DC converter 82 thus share the conductive tracks (804-810) in multiple conductive layers (L4-L10). As a result, a portion of the conductive tracks is shared by the power source wiring and charging wiring when heating and charging are not being performed simultaneously, thereby making it possible to reduce the size of the main board 50.

It should be noted that the conductive track 820 of the tenth conductive layer L10 serving as the negative electrode-side connecting portion 51b extends upward and is connected to ground wiring formed over a wide range across the tenth conductive layer L10 to the first conductive layer L1. The ground wiring will not be described.

By their nature, heating and charging involve a large current flow through wiring, so the power source wiring 510 during heating and the power source wiring 520 during charging are both preferably shortened. For this reason, both the heating elements, such as the step-up DC/DC converter 82 and the heating switches 85, 86, and the charging IC 81 constituting a charging element are preferably disposed close to the power source connecting portion 51, but in order to reduce the size of the main board 50, it is inevitable that either one of them will have to be set apart from the power source connecting portion 51.

Since there is a need to eliminate power loss during heating, it is preferable for the heating elements to be given priority in being arranged close to the power source connecting portion 51. Meanwhile, the charging IC 81 also preferably takes measurements close to the power source connecting portion 51 when measuring the power source voltage for controlling charging. That is to say, when the measurement point is remote from the power source, there is a proportional increase in wiring resistance and a drop in the accuracy of charging control.

Therefore, according to the present disclosure, the accuracy of measuring the power source voltage is improved by providing the charging IC 81, which is a charging element, with a power source voltage measurement pin Ps and acquiring a power source voltage measurement from close to the power source connecting portion 51 by way of voltage measurement wiring 521, rather than using the power source voltage acquired from the power source connection pins Pb of the charging IC 81, while the heating elements such as the step-up DC/DC converter 82 and the heating switches 85, 86 are arranged closer to the power source connecting portion 51 than the charging IC 81.

In more specific terms, the charging IC 81 is provided with the power source voltage measurement pin Ps, as shown in fig. 11. As shown in fig. 13, the power source voltage measurement pin Ps is connected to a signal track 521b of the seventh conductive layer L7 through a via 521a penetrating from the first conductive layer L1 to the sixth conductive layer L6. The signal track 521b extends downward from the via 521a, is connected to the conductive track 807, and is connected to the positive electrode-side connecting portion 51a (conductive track 810) through the conductive track 808 of the eighth conductive layer L8 and the conductive track 809 of the ninth conductive layer L9 which are formed at the same position. That is to say, the power source voltage measurement pin Ps of the charging IC 81 is connected to the conductive tracks 807-810 through the via 521a and the signal track 521b constituting the voltage measurement wiring 521.

Referring also to fig. 8, the power source voltage measurement pin Ps of the charging IC 81 is thus connected by way of the voltage measurement wiring 521 to a position closer to the power source connecting portion 51 than the power source connection pins Pb in the power source wiring 520 joining the positive electrode-side connecting portion 51a and the power source connection pins Pb. The charging IC 81 can therefore utilize the power source voltage at a position close to the positive electrode-side connecting portion 51a in the power source wiring 520, rather than utilizing the power source voltage input to the power source connection pins Pb of the charging IC 81. By this means, it is possible to improve the heating efficiency by arranging the heating elements such as the step-up DC/DC converter 82 and/or the heating switches 85, 86 closer to the power source connecting portion 51 than the charging IC 81, while the accuracy of charging control can also still be maintained with such an arrangement. Moreover, since the voltage measurement wiring 521 is wiring used for measurement, it does not require a large current flow, as is the case for the power source wiring 510, and fine wiring is therefore sufficient for the voltage measurement wiring 521, which can be installed without any increase in size.

It should be noted that in the embodiment described above, the signal track 521b was formed on the seventh conductive layer L7, which is a layer between the front surface 501 and the rear surface 502, but this is not limiting, and the signal track 521b may equally be provided on a layer closer to the positive electrode-side connecting portion 51a, e.g., the ninth conductive layer L9. In this case, the via 521a penetrates from the first conductive layer L1 to the eighth conductive layer L8. Furthermore, the signal track 521b is formed on the ninth conductive layer L9 and is connected to the conductive track 809 of the ninth conductive layer L9. That is to say, the power source voltage measurement pin Ps is connected to the signal track 521b of the ninth conductive layer L9 through the via 521a penetrating from the first conductive layer L1 to the eighth conductive layer L8. The signal track 521b extends downward from the via 521a, is connected to the conductive track 809, and is connected to the positive electrode-side connecting portion 51a (conductive track 810) of the tenth conductive layer L10 which is formed at the same position. That is to say, the power source voltage measurement pin Ps of the charging IC 81 is connected to the conductive tracks 809 and 810 through the via 521a and the signal track 521b constituting the voltage measurement wiring 521.

Furthermore, the signal track 521b may be provided on the tenth conductive layer L10. By forming the signal track 521b on a layer between the front surface 501 and the rear surface 502, the voltage measurement wiring 521 can be formed by utilizing an interface in a multilayer structure even if there is insufficient space on the element-mounting surface of the main board 50. Meanwhile, by providing the signal track 521b on the tenth conductive layer L10, it is possible to acquire the power source voltage at a position closer to the positive electrode-side connecting portion 51a. Furthermore, a signal track 521b may be provided on both a layer between the front surface 501 and the rear surface 502, and the layer on which the positive electrode-side connecting portion 51a is provided (the tenth conductive layer L10), the signal tracks 521b provided on these layers may be connected by vias, and the signal tracks 521b may be connected to the positive electrode-side connecting portion 51a on the front surface 501 (tenth conductive layer L10) on which the positive electrode-side connecting portion 51a is provided.

Furthermore, the step-up DC/DC converter 82 is provided with a feedback pin Pf for measuring an output voltage from the heating unit connection pin Po to the heating switch 85, as shown in fig. 8 and 10. In more specific terms, the feedback pin Pf of the step-up DC/DC converter 82 is connected to a signal track 515b on the second conductive layer L2 through a via 515a penetrating the first conductive layer L1, as shown in fig. 13. The signal track 515b extends obliquely downward from the via 515a toward the heating switch 85, and is connected to the heating switch 85 through a via 515c penetrating the first conductive layer L1. Here, among the plurality of vias in the wiring running from the heating unit connection pin Po to the heating switch 85, the via 515c is a via which is closer to the heating switch 85 than other elements. Moreover, capacitors C1-C3 shown in fig. 8 are examples of "other elements". The capacitors C1-C3 are each connected in parallel to the heating unit connection pin Po and a ground terminal of the step-up DC/DC converter 82. The capacitors C1-C3 are each electrically connected to the heating unit connection pin Po on the first conductive layer L1, and are also connected to vias 515d, 515e, 515f penetrating the first conductive layer L1. The vias 515d, 515e, 515f connect to ground.

Given that it is desirable for the power source voltage to be detected at a position as close as possible to the heater connecting portion 57a for purposes of feedback control, the step-up DC/DC converter 82 measures the output voltage through a via, among the plurality of vias in the wiring between the heating unit connection pin Po and the heating switch 85, which is closer to the heating switch 85 than other elements, and the heating voltage can therefore be controlled with greater accuracy. From this perspective, the feedback pin Pf preferably acquires the output voltage through the via among the plurality of vias which is closest to the heating switch 85. This makes it possible to control the heating voltage with even greater accuracy.

Although various embodiments of the present disclosure have been described above with reference to the drawings, it goes without saying that the present disclosure is not limited to such examples. It is obvious that a person skilled in the art will be able to conceive of a number of variant examples or modified examples within the scope disclosed in the claims, and any such variant examples or modified examples are naturally understood to fall within the technical scope of the present disclosure. Furthermore, the components in the embodiments described above may be combined in any way within a scope that does not depart from the essential point of the invention.

The present specification sets forth at least the following features. Note that corresponding components, etc. in the embodiment described above are shown in parentheses, but are not limited thereto.

(1) A power supply unit (power supply unit 110) for an aerosol-generating device (inhalation device 100B, 100), the power supply unit comprising: a power source (power source unit 111A-111C) for supplying power to a heating unit (heating unit 121A-121C) for heating an aerosol source (stick-type substrate 150);
   a power conversion device (step-up DC/DC converter 82) for converting power from the power source and supplying heating power to the heating unit and/or a heating switch (heating switch 85) for controlling power supply to the heating unit;
   a charging IC (charging IC 81) for receiving power from an external power source and performing control to supply charging power to the power source; and
   a board (main board 50) for mounting the charging IC, a power source connecting portion (power source connecting portion 51) supplied with power from the power source, and the power conversion device and/or the heating switch, wherein
   the charging IC comprises:
      a power source voltage measurement pin (power source voltage measurement pin Ps) for measuring a voltage of the power source; and
      a power source connection pin (power source connection pin Pb) to which the voltage of the power source is input,
      at least one of the power conversion device and/or the heating switch is disposed closer to the power source connecting portion than the charging IC, and
      the power source voltage measurement pin of the charging IC is connected by way of voltage measurement wiring (voltage measurement wiring 521) to a position closer to the power source connecting portion than the power source connection pin in power source wiring (power source wiring 520) joining the power source connecting portion and the power source connection pin.

According to (1), the heating elements such as the power conversion device and the heating switch are arranged close to the power source connecting portion, thereby enabling the power source wiring through which a large current flows to be shortened, which therefore makes it possible to reduce power loss and to improve heating efficiency. Meanwhile, although the charging IC is arranged further away than the heating elements because of space constraints, the power source voltage measurement pin of the charging IC is connected by way of voltage measurement wiring to a position closer to the power source connecting portion than the power source connection pin in power source wiring joining the power source connecting portion and the power source connection pin. The charging IC can therefore utilize the power source voltage at a position close to the power source connecting portion in the power source wiring through which a large current flows, rather than utilizing the power source voltage input to the power source connection pin. This makes it possible to maintain the accuracy of charging control while also improving heating efficiency. Moreover, since the voltage measurement wiring is wiring used for measurement, it does not require a large current flow, as is the case for the power source wiring, and fine wiring is therefore sufficient for the voltage measurement wiring, which can be installed without any increase in size.

(2) A power supply unit (power supply unit 110) for an aerosol-generating device (inhalation device 100B, 100), the power supply unit comprising: a power source (power source unit 111A-111C) for supplying power to a heating unit (heating unit 121A-121C) for heating an aerosol source (stick-type substrate 150);
a power conversion device (step-up DC/DC converter 82) for converting power from the power source and supplying heating power to the heating unit and/or a heating switch (heating switch 85) for controlling power supply to the heating unit;
a charging IC (charging IC 81) for receiving power from an external power source and performing control to supply charging power to the power source; and
a board (main board 50) for mounting the charging IC, a power source connecting portion (power source connecting portion 51) supplied with power from the power source, and the power conversion device and/or the heating switch, wherein
the charging IC comprises:
   a power source voltage measurement pin (power source voltage measurement pin Ps) for measuring a voltage of the power source; and
   a power source connection pin (power source connection pin Pb) to which the voltage of the power source is input,
   a wiring distance between at least one of the power conversion device and/or the heating switch and the power source connecting portion is shorter than a wiring distance between the charging IC and the power source connecting portion, and
   the power source voltage measurement pin of the charging IC is connected by way of voltage measurement wiring (voltage measurement wiring 521) to a position closer to the power source connecting portion than the power source connection pin in power source wiring (power source wiring 520) joining the power source connecting portion and the power source connection pin.

According to (2), the wiring distance between the heating elements, such as the power conversion device and the heating switch, and the power source connecting portion is shorter than the wiring distance between the charging IC and the power source connecting portion, so the power source wiring through which a large current flows can be shortened. This makes it possible to reduce power loss and to improve heating efficiency. Meanwhile, although the wiring distance between the charging IC and the power source connecting portion is longer because of space constraints, the power source voltage measurement pin of the charging IC is connected by way of the voltage measurement wiring to a position closer to the power source connecting portion than the power source connection pin in the power source wiring joining the power source connecting portion and the power source connection pin. The charging IC can therefore utilize the power source voltage at a position close to the power source connecting portion in the power source wiring through which a large current flows, rather than utilizing the power source voltage input to the power source connection pin. This makes it possible to maintain the accuracy of charging control while also improving heating efficiency. Moreover, since the voltage measurement wiring is wiring used for measurement, it does not require a large current flow, as is the case for the power source wiring, and fine wiring is therefore sufficient for the voltage measurement wiring, which can be installed without any increase in size.

(3) The power supply unit for an aerosol-generating device as disclosed in (1) or (2), wherein the board comprises:
a first face (rear surface 502) for mounting the power conversion device and/or the heating switch; and
a second face (front surface 501), on the opposite side to the first face, for mounting the power source connecting portion.

According to (3), by arranging the heating elements such as the power conversion device and the heating switch, and the power source connecting portion on different surfaces of the board, the heating elements and the power source connecting portion can be arranged closer together.

(4) The power supply unit for an aerosol-generating device as disclosed in (3), wherein the voltage measurement wiring includes: a via (via 521a) enabling communication between the first face and the second face; and wiring (signal track 521b) formed on at least one of the first face and the second face.

According to (4) voltage measurement wiring can be formed in a simple manner.

(5) The power supply unit for an aerosol-generating device as disclosed in (4), wherein
the voltage measurement wiring is connected to the power source connecting portion on the second face.

According to (5), it is possible to utilize the power source voltage at a position close to the power source connecting portion.

(6) The power supply unit for an aerosol-generating device as disclosed in (3), wherein
the board has a multilayer structure, and
the voltage measurement wiring includes wiring formed on a layer between the first face and the second face.

According to (6), the voltage measurement wiring can be formed by utilizing an interface in a multilayer structure even if there is insufficient space on the front surface of the board.

(7) The power supply unit for an aerosol-generating device as disclosed in any of (1)-(6), wherein
the power conversion device comprises:
a heating unit connection pin (heating unit connection pin Po) for supplying power to the heating unit via the heating switch; and
a feedback pin (feedback pin Pf) for measuring an output voltage from the heating unit connection pin to the heating switch,
a plurality of vias are provided in the wiring between the heating unit connection pin and the heating switch,
an element is electrically connected to any of the plurality of vias, and
the feedback pin acquires the output voltage through a via among the plurality of vias which is closer to the heating switch than the via to which the element is electrically connected.

According to (7), given that it is desirable for the power source voltage to be detected at a position as close as possible to the heating unit for purposes of feedback control, the power conversion device measures the output voltage through a via, among the plurality of vias in the wiring between the heating unit connection pin and the heating switch, which is closer to the heating switch than other elements, and the heating can therefore be controlled with greater accuracy.

(8) The power supply unit for an aerosol-generating device as disclosed in (7), wherein the element is a capacitor (capacitor C1-C3).

According to (8), it is possible to suppress a flow of an inrush current to the power conversion device.

(9) The power supply unit for an aerosol-generating device as disclosed in (8), wherein the capacitor is connected to the heating unit connection pin and to ground.

According to (9), it is possible to suppress a flow of an inrush current to the power conversion device.

(10) The power supply unit for an aerosol-generating device as disclosed in any of (1)-(9), wherein
the power conversion device comprises:
a heating unit connection pin (heating unit connection pin Po) for supplying power to the heating unit via the heating switch; and
a feedback pin (feedback pin Pf) for measuring an output voltage from the heating unit connection pin to the heating switch,
a plurality of vias are provided in the wiring between the heating unit connection pin and the heating switch,
the feedback pin acquires the output voltage through the via among the plurality of vias which is closest to the heating switch.

According to (10), given that it is desirable for the power source voltage to be detected at a position as close as possible to the heating unit for purposes of feedback control, the power conversion device measures the output voltage through the via, among the plurality of vias in the wiring between the heating unit connection pin and the heating switch, which is closest to the heating switch, and the heating can therefore be controlled with greater accuracy.

(11) The power supply unit for an aerosol-generating device as disclosed in any of (1)-(10), wherein
the power conversion device comprises:
a power source connection pin (power source connection pin Pb) to which the voltage of the power source is input,
the board has a multilayer structure, and
the power source wiring (power source wiring 520) joining the power source connecting portion and the power source connection pin of the charging IC, and power source wiring (power source wiring 510) joining the power source connecting portion and the power source connection pin of the power conversion device share wiring in multiple layers.

According to (11), a portion of the wiring is shared by the power source wiring and charging wiring when heating and charging are not being performed simultaneously, thereby making it possible to reduce the size of the board.

(12) The power supply unit for an aerosol-generating device as disclosed in any of (3)-(6), comprising an operational amplifier (operational amplifier 87) for measuring resistance of the heating unit,
wherein the operational amplifier is mounted on the first face.

According to (12) the amplifier prioritizes the signal-to-noise ratio, i.e., SNR, for measuring the heater resistance value over drift errors caused by heat generation, and the SNR is therefore increased by arranging the amplifier on the same surface as the heating elements.

(13) The power supply unit for an aerosol-generating device as disclosed in any of (3)-(6) and (12),
comprising a protection IC (protection IC 83) for protecting the power source,
wherein the protection IC is disposed on the second face.

According to (13), since the protection IC is prone to errors at high temperatures, a deterioration in control accuracy can be suppressed by mounting the protection IC on the opposite surface to the heating elements which are heat-generating elements.

(14) An aerosol-generating device (inhalation device 100A, 100B, 100) comprising: a heating unit (heating unit 121A-121C) for heating an aerosol source (stick-type substrate 150);
a power source (power source unit 111A-111C) for supplying power to the heating unit;
   a power conversion device (step-up DC/DC converter 82) for converting power from the power source and supplying heating power to the heating unit and/or a heating switch (heating switch 85) for controlling power supply to the heating unit;
a charging IC (charging IC 81) for receiving power from an external power source and performing control to supply charging power to the power source; and
a board (main board 50) for mounting the charging IC, a power source connecting portion (power source connecting portion 51) supplied with power from the power source, and the power conversion device and/or the heating switch, wherein
the charging IC comprises:
   a power source voltage measurement pin (power source voltage measurement pin Ps) for measuring a voltage of the power source; and
   a power source connection pin (power source connection pin Pb) to which the voltage of the power source is input,
   at least one of the power conversion device and the heating switch is disposed closer to the power source connecting portion than the charging IC, and
   the power source voltage measurement pin of the charging IC is connected by way of voltage measurement wiring (voltage measurement wiring 521) to a position closer to the power source connecting portion than the power source connection pin in power source wiring (power source wiring 520) joining the power source connecting portion and the power source connection pin.

According to (14), the heating elements such as the power conversion device and the heating switch are arranged close to the power source connecting portion, thereby enabling the power source wiring through which a large current flows to be shortened, which therefore makes it possible to reduce power loss and to improve heating efficiency. Meanwhile, although the charging IC is arranged further away than the heating elements because of space constraints, the power source voltage measurement pin of the charging IC is connected by way of voltage measurement wiring to a position closer to the power source connecting portion than the power source connection pin in power source wiring joining the power source connecting portion and the power source connection pin. The charging IC can therefore utilize the power source voltage at a position close to the power source connecting portion in the power source wiring through which a large current flows, rather than utilizing the power source voltage input to the power source connection pin. This makes it possible to maintain the accuracy of charging control while also improving heating efficiency. Moreover, since the voltage measurement wiring is wiring used for measurement, it does not require a large current flow, as is the case for the power source wiring, and fine wiring is therefore sufficient for the voltage measurement wiring, which can be installed without any increase in size.

### REFERENCE SIGNS LIST

50 Main board (board)
51 Power source connecting portion
81 Charging IC
82 Step-up DC/DC converter (power conversion device)
83 Protection IC
85 Heating switch
87 Operational amplifier (amplifier)
100A Inhalation device (aerosol-generating device)
100 Inhalation device (aerosol-generating device, power supply unit)
100B Inhalation device (aerosol-generating device, power supply unit)
110 Power supply unit
111A Power source unit (power source)
111B Power source unit (power source)
111C Power source unit (power source)
121A Heating unit
121B Heating unit
121C Heating unit
150 Stick-type substrate (aerosol source)
501 Front surface (second face)
502 Rear surface (first face)
510 Power source wiring (power source wiring joining power source connecting portion and power source connection pin of power conversion device)
520 Power source wiring (power source wiring joining power source connecting portion and power source connection pin of charging IC)
521 Voltage measurement wiring
521a Via
521b Signal track (wiring)
C1-C3 Capacitor
Pf Feedback pin
Ps Power source voltage measurement pin
Pb Power source connection pin
Po Heating unit connection pin

## Claims

1. A power supply unit for an aerosol-generating device, the power supply unit comprising: a power source for supplying power to a heating unit for heating an aerosol source;
a power conversion device for converting power from the power source and supplying heating power to the heating unit and/or a heating switch for controlling power supply to the heating unit;
a charging IC for receiving power from an external power source and performing control to supply charging power to the power source; and
a board for mounting the charging IC, a power source connecting portion supplied with power from the power source, and the power conversion device and/or the heating switch, wherein the charging IC comprises:
a power source voltage measurement pin for measuring a voltage of the power source; and
a power source connection pin to which the voltage of the power source is input,
at least one of the power conversion device and/or the heating switch is disposed closer to the power source connecting portion than the charging IC, and
the power source voltage measurement pin of the charging IC is connected by way of voltage measurement wiring to a position closer to the power source connecting portion than the power source connection pin in power source wiring joining the power source connecting portion and the power source connection pin.

2. A power supply unit for an aerosol-generating device, the power supply unit comprising: a power source for supplying power to a heating unit for heating an aerosol source;
a power conversion device for converting power from the power source and supplying heating power to the heating unit and/or a heating switch for controlling power supply to the heating unit;
a charging IC for receiving power from an external power source and performing control to supply charging power to the power source; and
a board for mounting the charging IC, a power source connecting portion supplied with power from the power source, and the power conversion device and/or the heating switch, wherein the charging IC comprises:
a power source voltage measurement pin for measuring a voltage of the power source; and
a power source connection pin to which the voltage of the power source is input,
a wiring distance between at least one of the power conversion device and/or the heating switch and the power source connecting portion is shorter than a wiring distance between the charging IC and the power source connecting portion, and
the power source voltage measurement pin of the charging IC is connected by way of voltage measurement wiring to a position closer to the power source connecting portion than the power source connection pin in power source wiring joining the power source connecting portion and the power source connection pin.

3. The power supply unit for an aerosol-generating device as claimed in claim 1 or 2, wherein the board comprises:
a first face for mounting the power conversion device and/or the heating switch; and
a second face, on the opposite side to the first face, for mounting the power source connecting portion.

4. The power supply unit for an aerosol-generating device as claimed in claim 3, wherein
the voltage measurement wiring includes: a via enabling communication between the first face and the second face; and wiring formed on at least one of the first face and the second face.

5. The power supply unit for an aerosol-generating device as claimed in claim 4, wherein
the voltage measurement wiring is connected to the power source connecting portion on the second face.

6. The power supply unit for an aerosol-generating device as claimed in claim 3, wherein
the board has a multilayer structure, and
the voltage measurement wiring includes wiring formed on a layer between the first face and the second face.

7. The power supply unit for an aerosol-generating device as claimed in any one of claims 1 to 6, wherein
the power conversion device comprises:
a heating unit connection pin for supplying power to the heating unit via the heating switch; and
a feedback pin for measuring an output voltage from the heating unit connection pin to the heating switch,
a plurality of vias are provided in the wiring between the heating unit connection pin and the heating switch,
an element is electrically connected to any of the plurality of vias, and
the feedback pin acquires the output voltage through a via among the plurality of vias which is closer to the heating switch than the via to which the element is electrically connected.

8. The power supply unit for an aerosol-generating device as claimed in claim 7, wherein the element is a capacitor.

9. The power supply unit for an aerosol-generating device as claimed in claim 8, wherein the capacitor is connected to the heating unit connection pin and to ground.

10. The power supply unit for an aerosol-generating device as claimed in any one of claims 1 to 9, wherein
the power conversion device comprises:
a heating unit connection pin for supplying power to the heating unit via the heating switch; and
a feedback pin for measuring an output voltage from the heating unit connection pin to the heating switch,
a plurality of vias are provided in the wiring between the heating unit connection pin and the heating switch,
the feedback pin acquires the output voltage through the via among the plurality of vias which is closest to the heating switch.

11. The power supply unit for an aerosol-generating device as claimed in any one of claims 1 to 10, wherein
the power conversion device comprises:
a power source connection pin to which the voltage of the power source is input,
the board has a multilayer structure, and
the power source wiring joining the power source connecting portion and the power source connection pin of the charging IC, and power source wiring joining the power source connecting portion and the power source connection pin of the power conversion device share wiring in multiple layers.

12. The power supply unit for an aerosol-generating device as claimed in any one of claims 3 to 6, comprising an operational amplifier for measuring resistance of the heating unit,
wherein the operational amplifier is mounted on the first face.

13. The power supply unit for an aerosol-generating device as claimed in any one of claims 3 to 6 and 12,
comprising a protection IC for protecting the power source,
wherein the protection IC is disposed on the second face.

14. An aerosol-generating device comprising: a heating unit for heating an aerosol source;
a power source for supplying power to the heating unit;
a power conversion device for converting power from the power source and supplying heating power to the heating unit and/or a heating switch for controlling power supply to the heating unit;
a charging IC for receiving power from an external power source and performing control to supply charging power to the power source; and
a board for mounting the charging IC, a power source connecting portion supplied with power from the power source, and the power conversion device and/or the heating switch, wherein
the charging IC comprises:
a power source voltage measurement pin for measuring a voltage of the power source; and
a power source connection pin to which the voltage of the power source is input,
at least one of the power conversion device and the heating switch is disposed closer to the power source connecting portion than the charging IC, and
the power source voltage measurement pin of the charging IC is connected by way of voltage measurement wiring to a position closer to the power source connecting portion than the power source connection pin in power source wiring joining the power source connecting portion and the power source connection pin.
